Europäisches Patentamt

(19) European Patent Office  (11) Publication number: **0 224 494**

Office européen des brevets  **B1**

(12) **EUROPEAN PATENT SPECIFICATION**

(45) Date of publication of patent specification: **23.01.91**  (51) Int. Cl.⁵: **A 61 F 13/15**

(21) Application number: **86902204.6**

(22) Date of filing: **19.03.86**

(86) International application number:
**PCT/US86/00564**

(87) International publication number:
**WO 86/05388 25.09.86 Gazette 86/21**

(54) **ABSORPTIVE PADS AND METHOD OF MAKING.**

(30) Priority: **20.03.85 US 714095**
**03.06.85 US 740217**

(43) Date of publication of application:
**10.06.87 Bulletin 87/24**

(45) Publication of the grant of the patent:
**23.01.91 Bulletin 91/04**

(84) Designated Contracting States:
**AT BE CH DE FR GB IT LI LU NL SE**

(56) References cited:
**EP-A-0 096 525**
**EP-A-2 083 748**
**US-A-2 134 930**
**US-A-3 121 427**

**THE JOURNAL OF INFECTIOUS DISEASES, vol. 151, no. 6, June 1985, pages 1158-1161, University of Chicago; J.T. MILLS et al.: "Control of production of toxic-shock-syndrome toxin-1 (TSST-1) by magnesium ion"**

(73) Proprietor: **PRESIDENT AND FELLOWS OF HARVARD COLLEGE**
**17 Quincy Street**
**Cambridge Massachusetts 02138 (US)**

(72) Inventor: **KASS, Edward, H.**
**Todd Pond Road**
**Lincoln, MA 01773 (US)**

(74) Representative: **Bizley, Richard Edward et al**
**BOULT, WADE & TENNANT 27 Furnival Street**
**London EC4A 1PQ (GB)**

## Description

This invention relates to catamenial tampons, surgical and wound dressings and packings, surgical sponges, absorbent materials for sanitary protection, and diapers, hereinafter collectively referred to as absorptive pads, and pertains more specifically to such pads effective to substantially inhibit increased production of toxic shock syndrome toxin-1 and other toxic staphylococcal products during use, and to the method of making such pads.

Absorptive pads comprising a mass of solid water-insoluble water-absorbent material formed into the desired configuration for application to or insertion into a wound or a body cavity and adapted for absorption and retention of body fluids have been used for many years. Cotton fibers and semi-synthetic fibers such as cellulose derivatives including acetate rayon, viscose rayon, polyacrylate rayon, and polyurethane, polyester and other fibers have been widely used in the manufacture of such absorptive pads as well as various non-fibrous water-absorbent materials, particularly cellulose derivatives such as carboxymethyl cellulose, synthetic polymers such as those water-swellable cross-linked polymers capable of forming hydrogels, polyurethane sponges, and others. While such absorptive pads provide high capacity absorption and retention characteristics, and hence provide extended periods of protection during use of the absorptive pads, such extended use has increased the time period during which possible bacterial growth and production of toxin can occur in the vicinity of the absorptive pad, particularly in the case of catamenial tampons. Toxic shock syndrome is a severe disease which has been found to be associated with toxic shock syndrome toxin-1, a staphylococcus-produced toxin, and has been associated with use of certain brands of tampons, as well as the use of certain nasal and other surgical packings.

It has previously been proposed in GB-A-2083748 to incorporate in hygienic napkins or diapers a soluble copper salt to inhibit growth of bacteria.

It has now been found that certain low concentrations of magnesium ion, below the concentration normally present in body fluids such as blood, are critical for optimal production of toxic shock syndrome toxin-1 and other staphylococcus products, some of which may be toxic, and it has also been found that various absorptive pad materials have the capability to absorb or bind magnesium ions.

The present invention provides a catamenial tampon comprising water-sorptive material having magnesium-binding capability and an amount of non-toxic water-soluble salt of a magnesium cation at least sufficient to satisfy the magnesium-binding capacity of the tampon and effective to substantially inhibit production of toxic shock syndrome toxin-1 during use of said tampon. The invention also provides the method of making a catamenial tampon comprising water-sorptive material having magnesium binding capability which comprises including with said material an amount of a non-toxic water-soluble salt of magnesium cation at least sufficient to satisfy the magnesium binding capacity of said material, and forming said material into a tampon.

Any non-toxic magnesium salt can be used provided it is sufficiently water-soluble to provide the desired quantity of divalent cations to saturate the magnesium absorbing or binding capacity of the absorptive pad material; the absorbing or binding capacity varies widely depending upon the identity of the particular materials present in a pad as well as upon the manufacturing procedures followed in each case. In the case of polacrylate rayon fibers, for example, 1000 micrograms of magnesium acetate or even less per gram of fiber suffices. In general, an amount of magnesium salt effective to inhibit production of toxic shock syndrome toxin-1 or other toxic staphylococcal product during the use of an absorptive pad to a level no greater than that normally present in the absence of a pad will be sufficient to satisfy the magnesium absorbing or binding capacity of the pad. The anionic portion of the salt is not critical and may vary widely since very low solubility in water suffices for the purpose of the present invention. Suitable salts include the magnesium chloride, sulfate, nitrate, acetate, palmitate, stearate, mandelate, hippurate, and the like. Magnesium stearate and magnesium acetate are particularly preferred.

The salt may be incorporated in the tampon by immersing the completed pad in an aqueous solution or dispersion of the salt, but it is usually more convenient and effective to treat one or more of the component materials with the desired salt at some point in the manufacturing procedure before or during incorporation of the material in the pad. For example, in the case of synthetic fiber materials, the fibers can be passed through an aqueous bath containing a suitable salt, or the fibers can be passed through a pair of squeeze rolls to which a supply of salt solution is supplied. Magnesium stearate is a preferred salt for application to synthetic fibers because it facilitates processing of the fibers. It is also possible to incorporate the desired salt in a portion only of the pad, for example in the outer wrapper, so that it is in position to be distributed or diffused under conditions of use to those other portions of the pad which require saturation of their magnesium absorbing or binding capacity. It is preferred, however, that the magnesium absorbing or binding capacity of all components of the pad be satisfied initially, before use of the pad.

An excess of salt above the amount required to saturate the magnesium absorbing or binding capacity of the absorbent material is not harmful, and there is no critical upper limit on the amount of salt employed in order to inhibit production of the toxin.

While it is not intended to limit this invention to any particular theory or mode of operation, it is believed that one or more of the water-sorptive solid materials normally present in pads such as tampons possesses sufficient absorptive or specific binding capacity for magnesium ions to reduce the concentration of such ions normally present in body fluids to a critical low level at which production of toxic shock syndrome toxin-1 and other toxic staphylococcal products is greatly enhanced, and that the present invention operates by eliminating this capacity.

## Claims

1. A catamenial tampon comprising water-sorptive material having magnesium-binding capability and an amount of non-toxic water-soluble salt of a magnesium cation at least sufficient to satisfy the magnesium-binding capacity of the tampon and effective to substantially inhibit production of toxic shock syndrome toxin-1 during use of said tampon.

2. A tampon as claimed in claim 1 in which said salt is magnesium stearate or magnesium acetate.

3. A method of making a catamenial tampon comprising water-sorptive material having magnesium binding capability which comprises including with said material an amount of non-toxic water-soluble salt of magnesium cation at least sufficient to satisfy the magnesium binding capacity of said material, and forming said material into a tampon.

4. A method as claimed in claim 3 in which said salt is magnesium stearate or magnesium acetate.

5. The use of a magnesium cation in the manufacture of a catamenial tampon in an amount at least sufficient to satisfy the magnesium-binding capacity of the tampon and sufficient to substantially inhibit the production of staphylococcal products during use of said tampon.

## Patentansprüche

1. Katamenien-Tampon, umfassend ein für Wasser aufnahmefähiges Material mit der Fähigkeit, Magnesium zu binden, und eine Menge eines nicht-toxischen wasserlöslichen Salzes eines Magnesium-Kations, die wenigstens ausreicht, um das Bindevermögen des Tampons für Magnesium zu befriedigen, und dahingehend wirksam ist, daß sie während des Gebrauchs des Tampons die Erzeugung des toxischen Schock-Syndrom-Toxins-1 im wesentlichen hemmt.

2. Tampon nach Anspruch 1, worin das Salz Magnesiumstearat oder Magnesiumacetat ist.

3. Verfahren zur Herstellung eines ein für Wasser aufnahmefähiges Material mit der Fähigkeit, Magnesium zu binden, umfassenden Katamenien-Tampons, umfassend das Einarbeiten einer Menge eines nicht-toxischen wasserlöslichen Salzes eines Magnesium-Kations, die wenigstens ausreicht, um das Bindevermögen des Materials für Magnesium zu befriedigen, in dieses Material und das Formen eines Tampons aus diesem Material.

4. Verfahren nach Anspruch 3, worin das Salz Magnesiumstearat oder Magnesiumacetat ist.

5. Verwendung eines Magnesium-Kations bei der Fertigung eines Katamenien-Tampons in einer Menge, die wenigstens ausreicht, um das Bindevermögen des Tampons für Magnesium zu befriedigen, und die ausreicht, um während des Gebrauchs des Tampons die Erzeugung von Staphylokokken-Produkten im wesentlichen zu hemmen.

## Revendications

1. Tampon périodique comprenant une matière présentant la capacité de sorption de l'eau, capable de fixer le magnésium, et une quantité d'un sel hydrosoluble non toxique d'un cation magnésium au moins suffisante pour satisfaire la capacité de fixation du magnésium du tampon et efficace pour inhiber fortement la production de la toxine-1 du syndrome de choc toxique au cours de l'utilisation dudit tampon.

2. Tampon suivant la revendication 1, dans lequel le sel est le stéarate de magnésium ou l'acétate de magnésium..

3. Procédé de production d'un tampon périodique comprenant une matière présentant la capacité de sorption de l'eau, capable de fixer le magnésium, qui consiste à incorporer à ladite matière une quantité d'un sel hydrosoluble non toxique d'un cation magnésium, au moins suffisante pour satisfaire la capacité de fixation du magnésium de ladite matière, et à mettre ladite matière sous forme d'un tampon.

4. Procédé suivant la revendication 3, dans lequel le sel est le stéarate de magnésium ou l'acétate de magnésium.

5. Utilisation dans la production d'un tampon périodique d'un cation magnésium en une quantité au moins suffisante pour satisfaire la capacité de fixation du magnésium du tampon et suffisante pour inhiber fortement la formation de produits staphylococciques au cours de l'utilisation dudit tampon.